(19) **Europäisches Patentamt**

**European Patent Office**

**Office européen des brevets**

(11) **EP 0 925 780 B1**

(12) **FASCICULE DE BREVET EUROPEEN**

(45) Date de publication et mention de la délivrance du brevet:
**03.07.2002 Bulletin 2002/27**

(51) Int Cl.⁷: **A61K 7/48**, A61K 7/021

(21) Numéro de dépôt: **98402996.7**

(22) Date de dépôt: **30.11.1998**

(54) **Composition cosmétique sans transfert comprenant une dispersion de particules de polymère dans une phase grasse liquide**

Nichtübertragende kosmetische Zusammensetzungen die eine Dispersion von Polymerteilchen in einer flüssigen Fettphase enthält

Non-transfer cosmetic composition comprising a dispersion of polymer particles in a liquid fatty phase

(84) Etats contractants désignés:
**DE ES FR GB IT**

(30) Priorité: **22.12.1997 FR 9716251**

(43) Date de publication de la demande:
**30.06.1999 Bulletin 1999/26**

(73) Titulaire: **L'OREAL**
**75008 Paris (FR)**

(72) Inventeurs:
• **de la Poterie, Valérie**
**77820 Le Chatelet en Brie (FR)**

• **Mougin, Nathalie**
**75011 Paris (FR)**

(74) Mandataire: **Lhoste, Catherine**
**L'OREAL-DPI**
**6 rue Bertrand Sincholle**
**92585 Clichy Cédex (FR)**

(56) Documents cités:
**EP-A- 0 749 746     WO-A-97/01321**
**FR-A- 2 232 302     FR-A- 2 232 303**

**Description**

La présente invention a trait à une composition contenant des particules de polymère dispersées dans une phase grasse, destinée en particulier aux domaines cosmétique, dermatologique, pharmaceutique et hygiénique. Plus spécialement, l'invention se rapporte à une composition sans transfert pour le soin et/ou le maquillage de la peau aussi bien du visage que du corps humain, des muqueuses comme les lèvres et l'intérieur des paupières inférieures, ou encore des phanères comme les cils, les sourcils, les ongles et les cheveux.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyes liners, les compositions de protection solaire ou de coloration de la peau.

Les produits de maquillage ou de soin de la peau ou des lèvres des êtres humains comme les fonds de teint ou les rouges à lèvres contiennent généralement des phases grasses telles que des cires et des huiles, des pigments et/ou charges et, éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

Depuis plusieurs années, les cosméticiens se sont intéressés aux compositions de rouge à lèvres et plus récemment aux compositions de fond de teint "sans transfert". Ainsi, la société Shiseido a envisagé dans sa demande de brevet JP-A-61-65809 des compositions de rouge à lèvres "sans transfert" contenant une résine siloxysilicate (à réseau tridimensionnel), une huile de silicone volatile à chaîne silicone cyclique et des charges pulvérulentes. De même la société Noevier à décrit dans le document JP-A-62-61911 des compositions de rouge à lèvres, d'eye liner, de fonds de teint "sans transfert" comportant une ou plusieurs silicones volatiles associées à une ou plusieurs cires hydrocarbonées.

Ces compositions, bien que présentant des propriétés de "sans transfert" améliorées ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres. Pour améliorer le confort de ce type de composition, on pourrait ajouter des huiles non volatiles siliconées ou non. mais dans ce cas on perd en efficacité "sans transfert".

Plus récemment, la société Procter & Gamble a envisagé dans sa demande de brevet WO-A-96/36323 des compositions de mascara de type émulsion eau-dans-huile présentant une longue tenue, une résistance à l'eau et ne laissant pas de traces. Ces compositions contiennent, entre autre, un polymère insoluble dans l'eau, appelé généralement un latex, associé à un tensioactif du type alkyl ou alcoxy diméthicone copolyol, des huiles hydrocarbonées, des pigments et charges ainsi que des cires.

Les compositions à base d'huiles de silicones et de résines siliconées ainsi que celles à base de latex conduisent à des films colorés mats. Or, la femme est aujourd'hui à la recherche de produits notamment de coloration des lèvres, brillants. De plus, les propriétés de sans transfert des films déposés ne sont pas parfaites. En particulier, une pression ou un frottement prononcé, conduit à une diminution de la couleur du dépôt et à un redépôt sur le support mis en contact avec ces films..

En outre, les documents EP-A-497144 et FR-A-2 357 244 décrivent des compositions dites "sans transfert", contenant un polymère bloc styrène-éthylène-propylène associé à des cires, des huiles légères ou volatiles et des pigments. Ces compositions présentent l'inconvénient d'être peu confortables, d'avoir des propriétés cosmétiques quelconques et d'être difficilement formulables. Par ailleurs, les propriétés "sans transfert" de ces compositions sont très moyennes.

Il subsiste donc le besoin d'une composition ne présentant pas les inconvénients ci-dessus, et ayant notamment des propriétés de "sans transfert" total, même lors d'une pression ou d'un frottement prononcé ou intensif, un aspect plus ou moins brillant, adapté au désir de la consommatrice, ne desséchant pas au cours du temps la peau ou les lèvres sur lesquelles elle est appliquée.

La demanderesse a constaté, de façon tout à fait surprenante, que l'utilisation de particules de polymère dispersées dans une phase grasse, dans une composition cosmétique, dermatologique, pharmaceutique ou hygiénique pouvait permettre d'obtenir un film brillant, de très bonne tenue, ne transférant pas du tout, résistant à l'eau, tout en étant très agréable à l'application et à porter tout au long de la journée. Le film est notamment souple, flexible et non collant.

La présente invention a donc pour objet une composition à application topique, telle que définie dans la revendication 1.

Cette composition est en particulier une composition cosmétique, dermatologique, hygiénique ou pharmaceutique. Elle contient donc des ingrédients compatibles avec la peau, les muqueuses et les fibres kératiniques ou phanères.

Le ou les polymères utilisés dans la présente invention peuvent être de toute nature. On peut ainsi employer un polymère radicalaire, un polycondensat, voire un polymère d'origine naturelle et leurs mélanges. Le ou les polymères peuvent être choisis par l'homme du métier en fonction de ses propriétés et selon l'application ultérieure souhaitée pour la composition. Ainsi, le polymère peut être filmifiable ou non. Cependant, l'obtention d'un film totalement "sans transfert" est plus spécialement dû à l'utilisation d'un polymère filmifiable.

Par "phase grasse volatile", on entend tout milieu non aqueux susceptible de s'évaporer de la peau ou des lèvres, en moins d'une heure.

Un autre objet de l'invention est l'utilisation dans une composition cosmétique ou pour la fabrication d'une composition pharmaceutique à application topique telle que définie dans la revendication 27.

Un autre objet de l'invention est l'utilisation dans une composition cosmétique ou pour la fabrication d'une composition pharmaceutique à application topique telle que définie dans la revendication 28.

L'invention a encore pour objet un procédé de soin cosmétique ou de maquillage des lèvres ou de la peau, consistant à appliquer sur respectivement les lèvres ou la peau une composition cosmétique telle définie précédemment.

L'invention a encore pour objet un procédé cosmétique pour limiter, voire supprimer, le transfert d'une composition de maquillage ou de soin cosmétique de la peau ou des lèvres sur un support différent de la dite peau et desdites lèvres, tel que définl dans la revendication 30.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

On a de plus constaté que les compositions selon l'invention, présentent des qualités d'étalement et d'adhésion sur la peau, les semi-muqueuses ou les muqueuses, particulièrement intéressantes, ainsi qu'un toucher onctueux et agréable. Ces compositions ont, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

Les compositions selon l'invention comprennent donc avantageusement une dispersion stable de particules généralement sphériques d'au moins un polymère, dans une phase grasse liquide physiologiquemént acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés mécaniques de la compositions en fonction de l'application envisagée.

Il est possible d'utiliser des polymères filmifiables, de préférence ayant une (Tg) basse, inférieure ou égale à la température de la peau. On obtient ainsi une dispersion qui peut filmifier lorsqu'elle est appliquée sur un support, ce qui n'est pas le cas lorsque l'on utilise des dispersions de pigments minéraux selon l'art antérieur.

Les polymères utilisables dans la composition de l'invention ont de préférence un poids moléculaire de l'ordre de 2000 à 10 000 000 et une (Tg) de -100°C à 300°C.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuel lement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à 40°C et notamment les acrylates de méthyle éventuellement copolymérisés avec l'acide acrylique.

Parmi les polymères non filmifiables, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

Le phase grasse liquide dans laquelle est dispersé le polymère, peut être constituée de toute huile cosmétiquement ou dermatologiquement acceptable, et de façon générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante.

On peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante et pression atmosphérique ayant par exemple une tension de vapeur à pression et température ambiantes non nulle et en particulier allant de $10^{-3}$ à 300 mm de Hg à condition que la température d'ébullition soit supérieure à 30°C. Ces huiles volatiles sont favorables à l'obtention d'un film à propriétés "sans transfert" total. Après évaporation de ces huiles, on obtient un dépôt filmogène souple, non collant sur la peau ou les muqueuses. suivant respectivement le mouvements de la peau ou des lèvres, sur lesquelles la composition est appliquée. Ces huiles volatiles facilitent, en outre, l'application de la composition sur la peau, les muqueuses, les phanères.

Ces huiles peuvent être des huiles hydrocarbonées, des huiles silicones comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone ainsi que les isoparaffines en $C_8$-$C_{15}$. Ces huiles volatiles représentent notamment de 30 à 97,99 % du poids total de la composition, et mieux de 30 à 75 %.

Comme huile volatile utilisable dans l'invention, on peut citer notamment l'octaméthylcyclotétrasiloxane, le décaméthylcyclopentasiloxane, l'hexadécaméthylcyclohexasiloxane. l'heptaméthylhexyltrisiloxane, l'heptaméthyloctyltrisiloxane ou les isoparaffines en $C_8$-$C_{16}$ telles que les 'ISOPARs', les PERMETYLs et en particulier l'isododécane.

Dans un mode particulier de réalisation de l'invention, on choisit le phase grasse liquide dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global $\delta$ global selon l'espace de solubilité de HANSEN est défini dans l'article "Solubility parameter values" de Eric A. Grulke de l'ouvrage "Polymer Handbook" 3$^{eme}$ édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).
La définition des solvants dans l'espace de solubilité tridimensionnel selon HANSEN est décrite dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN inférieur ou égal à 17 $(MPa)^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère et/ou de la nature du stabilisant, comme indiqué ci-après.

De plus, la phase grasse liquide dans laquelle est dispersé le polymère peut représenter de 30 % à 97,99 % du poids total de la composition et de préférence de 30 à 75 %.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée : les polymères hydrocarbonés greffés avec une chaîne siliconée.

Conviennent également les copolymères greffés ayant par exemple un squelette insoluble de type polyacrylique avec des greffons solubles de type acide polyhydroxystéarique : copolymères à base d'acrylates ou de méthacrylates d'alcools en $C_1$-$C_4$, et d'acrylates ou de méthacrylates d'alcools en $C_8$-$C_{30}$.

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

Comme copolymères blocs greffés ou séquencés comprenant au moins un bloc de type polyorganosiloxane et au moins d'un polyéther, on peut utiliser les copolyol diméthicones te!s que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés comprenant au moins un bloc résultant de la polymérisation de monomère à liaison(s) éthylénique(s) simples ou conjuguées, et au moins un bloc d'un polymère vinylique, on peut citer les copolymères séquencés. notamment de type "dibloc" ou "tribloc" du type polystyrène/polyisoprène, polystyrène/ polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation de monomère à liaison(s) éthylénique(s) (dénommé souvent dans la littérature par diène, hydrogéné ou non hydrogéné), et au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation de monomère à liaison(s) éthylénique(s), et au moins un bloc d'un polyéther, on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères d'acrylates ou de méthacrylates d'alcools en $C_1$-$C_4$, et d'acrylates ou de méthacrylates d'alcools en $C_8$-$C_{30}$. On peut en particulier citer le copolymère méthacrylate de stéaryle/méthacrylate de méthyle.

De préférence, on choisit en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

D'autre part, lorsque le phase grasse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester.

Lorsque le phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alcools en $C_1$-$C_4$, et d'acrylates ou de méthacrylates d'alcools en $C_9$-$C_{30}$,

- (c) les copolymères blocs greffés ou séquencés comprenant au moins un bloc résultant de la polymérisation de diène, hydrogéné ou non hydrogéné.

et au moins un bloc d'un polymère vinylique ou acrylique ou d'un polyéther ou d'un polyester, ou leurs mélanges.

Les dispersions obtenues selon l'invention peuvent alors être utilisées dans une composition notamment cosmétique, dermatologique, pharmaceutique et/ou hygiénique, telle qu'une composition de soin ou de maquillage de la peau ou des lèvres, ou encore une composition capillaire ou une composition solaire ou de coloration de la peau.

Suivant l'application, on pourra choisir d'utiliser des dispersions de polymères filmifiables ou non filmifiables, dans des huiles volatiles ou non volatiles.

La composition peut comprendre comme matière colorante contenant un ou plusieurs composés pulvérulents et/ou un ou plusieurs colorants liposolubles, par exemple à raison de 0,01 à 70% du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 40 % du poids total de la composition et mieux de 1 à 30 %. Plus la quantité de composés pulvérulents diminue, plus les qualités de sans transfert et de confort augmentent. Le fait que les propriétés de sans transfert augmentent au fur et à mesure que la quantité de composés pulvérulents diminue est tout à fait surprenant. En effet, jusqu'à ce jour les propriétés de sans transfert des compositions de l'art antérieur augmentaient avec la quantité de composés pulvérulents. Inversement, leurs inconforts et leur sécheresse sur la peau ou muqueuses augmentaient.

Par ailleurs, la propriété de sans transfert augmente avec la quantité de polymère dispersible dans la phase grasse liquide. En pratique, le polymère peut représenter en matière active jusqu'à 60 % (en matière active ou sèche) du poids total de la composition. En utilisant au-dessus de 12 % en poids de matière active de polymère dans la composition et jusqu'à 60 %, on obtient un film sans transfert total. Entre 2 % et 12 % l'effet sans transfert est notable sans toutefois être total. On peut donc adapter les propriétés sans transfert à volonté, ce qui n'était pas possible avec les compositions sans transfert de l'art antérieur, sans nuire au confort du film déposé.

De façon préférentielle, le rapport en poids de pigment(s)/polymère est < 1 et même ≤ 0,9. De préférence, ce rapport est ≤ 0,5. Ce rapport peut descendre jusqu'à 0,015. Au dessus de 0,5, le film transfert légèrement et au dessus de 1 le film transfert de façon importante. En particulier les inventeurs ont fait varier le taux de pigments et de polymère. Les résultats sont donnés dans le tableau 1 ci-après.

### TABLEAU 1

| Composition | Polymère de l'exemple 1 | Isododécane | Pigment | Pig/polymère | Transfert |
|---|---|---|---|---|---|
| A | 1 | 98 | 1 | 1 | un peu |
| B | 2 | 97 | 1 | 0,5 | non |
| C | 5 | 94 | 1 | 0,2 | non |
| D | 7 | 92 | 1 | 0,14 | non |
| E | 10 | 89 | 1 | 0,1 | non |
| F | 1 | 96 | 3 | 3 | oui |
| G | 1 | 94 | 5 | 5 | oui |
| H | 2 | 95 | 3 | 1,5 | oui |
| I | 5 | 92 | 3 | 0,6 | oui léger |
| J | 6 | 91 | 3 | 0,5 | non |

La composition de l'invention peut comprendre, avantageusement, au moins 30 % en poids de phase grasse, par rapport au poids total de la composition. En dessous de 30 %. on obtient une texture granuleuse et pulvérulente. Ceci est peu souhaitable lorsque l'on cherche à obtenir un aspect crémeux, gélifié ou en stick, homogène non granuleux.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore. les poudres de polymères de tétrafluoroéthyléne, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0,01 à 20 % du poids de la compositions et mieux de 0,1 à 6 %.

Le polymère de la composition de l'invention permet la formation d'un film sur la peau, les lèvres et/ou les muqueuses, formant un réseau piégeant les matières colorantes et/ou les actifs. Selon la quantité relative de matières colorantes, utilisée par rapport à la quantité de polymère stabilisé, utilisée, il est possible d'obtenir un film plus ou moins brillant et plus ou moins sans transfert.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme et notamment à des concentrations de 0,001 à 20 % du poids total de la composition.

La composition selon l'invention peut, de plus, comprendre, selon le type d'application envisagée, les constituants classiquement utilisées dans les domaines considérés, qui sont présents en une quantité appropriée à la forme galénique souhaitée.

En particulier, elle peut comprendre, outre, la phase grasse liquide dans laquelle le polymère est stabilisé des phases grasses additionnelles qui peuvent être choisies parmi les cires, les huiles, les gommes et/ou les corps gras pâteux, d'origine végétale, animale, minérale ou de synthèse, voire siliconé, et leurs mélanges

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice". L. M. Prince Ed., Academic Press (1977), pages 21-32. Comme cire liquide à température ambiante, on peut citer l'huile de Jojoba.

Les cires peuvent être présentes à raison de 0-50% en poids dans la composition et mieux de 10 à 30 %.

La composition peut comprendre, en outre, tout additif usuellement utilisé dans de telles compositions, tel que des épaississants, des antioxydants, des parfums, des conservateurs, des tensioactifs, des polymères liposolubles comme les polyalkylènes, notamment le polybutène, les polyacrylates et les polymères siliconés compatibles avec la phase grasse ainsi que les dérivés de polyvinylpyrolidone. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple, de viscosité dynamique à 25°C de l'ordre de 1 à 40 Pa.s ou encore de gel, de crème plus ou moins fluide. Elles peuvent alors constituées des fonds de teint ou des rouges à lèvres, des produits solaires ou de coloration de la peau.

Les compositions de l'invention sont avantageusement anhydres et peuvent contenir moins de 5 % d'eau par rapport au poids total de la composition. Elles peuvent alors se présenter notamment sous forme de gel huileux, de liquide huileux ou huile, de pâte ou de stick ou encore sous forme de dispersion vésiculaire contenant des lipides ioniques et/ou non ioniques. Ces formes galéniques sont préparées selon les méthodes usuelles des domaines considérés.

Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple crème de soin anhydre, huile solaire, gel corporel), une composition de maquillage (par exemple gel de maquillage) ou une composition de bronzage artificiel.

L'invention est illustrée plus en détail dans les exemples suivants.

**Exemple 1 de dispersion de polymère**

On prépare une dispersion de copolymère non réticulé d'acrylate de méthyle et d'acide acrylique dans un rapport 85/15, dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éhylène-propylène) vendu sous le nom de KRATON G1701 (Shell), ayant un taux de matière sèche de 22,6% en poids et une taille moyenne des particules de 175 nm (polydispersité : 0,05) et une Tg de 20°C. Ce copolymère est filmifiable.

**Exemple 2 de « rouge » à lèvres**

On prépare un « rouge» à lèvres sous forme fluide ayant la composition suivante :

- dispersion selon l'exemple 1        90.7 g
- huile de parléam        2,1 g
- octyldodécanol        0,9 g
- PVP/eicosène        1,2 g
- phényltriméthicone        2,1 g
- pigments        3 g

Les pigments renferme un mélange de DC Red 27, DC Red 7, DC Red 36, d'oxyde de fer noir et d'oxyde de fer brun. Le rapport pigments/polymère est de 0,15.

La composition est préparée par simple mélange à température ambiante des différents constituants, après broyage des pigments dans les huiles. On obtient un « rouge » à lèvres facile à appliquer, et qui permet l'obtention d'un film confortable, souple et non collant. Ce film est, en outre, brillant et "sans transfert" total. Il résiste parfaitement bien à l'eau et se démaquille avec une huile démaquillante classique.

Un test sensoriel a été effectué avec ce « rouge » à lèvres sur plusieurs personnes. Le test de sans transfert a été réalisé dans les conditions suivantes : application du produit sur les lèvres, séchage à l'air libre pendant 2 minutes puis application des lèvres sur un papier filtre. Ce test est répété dans les même conditions avec un temps de séchage de 10 minutes. Le sans transfert est jugé comme ayant une efficacité de 98 %.

De plus les personnes du test de sans transfert ont jugé le produit facile à étaler, conférant un maquillage homogène et adhérent, très couvrant et de couleur prononcée. Le contour des lèvres est net. La texture du produit est jugé fluide et agréable à l'application. Le démaquillage s'est effectué avec un démaquillant classique (Bifacil de Chez Lancôme) sans laisser de traces.

**Exemple 3 de fond de teint**

On prépare la composition suivante :

- dispersion de l'exemple 1 (22.6 % de matière sèche)        82,0 g
- poudre de Nylon        8,0 g
- oxyde de fer jaune        1.1 g
- oxyde de fer brun-jaune        0,6 g
- oxyde de fer noir        0,3 g
- Oxyde de titane        8,0 g

Le rapport pigments/polymère est de 0,55.

On obtient un fond de teint qui peut être appliqué sur le corps, notamment sur le cou, et le visage. Le maquillage est naturel, mat, résistant à l'eau et présente de très bonnes propriétés de sans transfert.

**Exemple 4 de rouge à lèvres**

On prépare un rouge à lèvres sous forme de stick ayant la composition suivante :

- .   dispersion de polymère (*) 4        8,3 g
- .   huile de parléam        7 g
- .   octyldodécanol        3 g
- .   PVP/eicosène        4 g
- .   DC Red 27        2,2 g
- .   phényltriméthicone        7,0 g
- .   DC Red 7        4,2 g
- .   DC Red 36        1.12 g
- .   oxyde de fer noir        0,08 g
- .   oxyde de fer brun        2.4 g
- .   cire de polyéthylène (Poly wax 500)        20,7 g

Le polymère est préparé selon l'exemple 1 avec 95 % d'acrylate de méthyle et 5 % d'acide acrylique. Le rapport pigments/polymère est de 0.9.

La composition est préparée comme suit : broyage des pigments dans les huiles chauffées légèrement ; ajout de la cire de polyéthylène à 100 °C ; léger refroidissement puis ajout de la dispersion de polymère et enfin coulage dans un moule approprié pou former un stick de rouge à lèvres.

Un test sensoriel a été effectué avec ce rouge à lèvres sur plusieurs personnes en comparaison d'un rouge à lèvres de l'état de la technique (Colour Endure de L'oréal). Le test de sans transfert a été réalisé dans les conditions suivantes : application du produit sur les lèvres, séchage à l'air libre pendant 2 minutes puis application des lèvres sur un papier filtre. Ce test est répété dans les même conditions avec un temps de séchage de 10 minutes.

L'application des 2 produits est aussi facile. Le geste de maquillage est plus précis avec le stick de l'invention car le produit est plus rigide. Le maquillage est jugé homogène pour les 2 produits, plus vivant et plus brillant avec le stick de l'invention. Il est non collant pour les 2 produits, de sensation légère et ne procure pas de tiraillement. Le transfert est plus discret avec le stick de l'invention, sachant que le produit de l'art antérieur avait déjà de très bonnes propriétés de sans transfert. Le démaquillage est facile pour les 2 produits et ne laisse pas de trace sur les lèvres.

**Revendications**

1.   Composition à application topique, comprenant une phase grasse liquide et au moins une matière colorante, **caractérisée par le fait qu'**elle comprend, de plus, au moins 2 % en poids, par rapport au poids total de la composition, de particules de polymère filmifiable dispersées et stabilisées en surface par au moins un stabilisant, dans ladite phase grasse liquide, que cette phase grasse contient au moins une huile volatile à température ambiante, ayant une tension de vapeur à température ambiante et pression atmosphériques allant de $10^{-3}$ à 300 mm de Hg à condition que la température d'ébullition soit supérieure à 30°C et que la matière colorante contient des pigments dans un rapport pigment(s)/polymère inférieur à 1.

2.   Composition selon la revendication 1, **caractérisée en ce que** la phase grasse liquide est cosmétique, dermatologique, hygiénique ou pharmaceutique.

3.   Composition selon l'une des revendications précédentes, dans laquelle le polymère est choisi parmi les polymères radicalaires, les polycondensats, les polymères d'origine naturelle et leurs mélanges.

4.   Composition selon l'une des revendications précédentes, dans laquelle le polymère est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

5.   Composition selon l'une des revendications précédentes, dans laquelle la phase grasse liquide est constitué d'huiles d'origine minérale, animale, végétale ou synthétique, carbonées, hydrocarbonées, fluorées et/ou siliconées, seules ou en mélange.

**6.** Composition selon l'une des revendications précédentes, dans laquelle la phase grasse liquide contient une huile choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'Isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool olélque, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

**7.** Composition selon l'une des revendications précédentes, dans laquelle la phase grasse liquide est choisie dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

**8.** Composition selon l'une des revendications précédentes, dans laquelle le stabilisant est choisi parmi les polymères séquencés, les polymères greffés, les polymères statistiques et leurs mélanges.

**9.** Composition selon l'une des revendications précédentes, dans laquelle le stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide polyhydroxystéarique ; les copolyméres blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alcools en $C_1$-$C_4$, ou d'acrylates ou de méthacrylates d'alcools en $C_8$-$C_{30}$; les copolymères blocs greffés ou séquencés comprenant au moins un bloc résultant de la polymérisation de monomère à liaison(s) éthylénique(s) et au moins un bloc d'un polymère vinylique ; les copolymères blocs greffés ou séquencés comprenant au moins un bloc résultant de la polymérisation de monomère à liaison(s) éthylénique(s) et au moins un bloc d'un polymère acrylique ; les copolymères blocs greffés ou séquencés comprenant au moins un bloc résultant de la polymérisation de monomère à liaison(s) éthylénique(s) et au moins un bloc d'un polyéther.

**10.** Composition selon l'une des revendications précédentes, **caractérisée en ce que** le stabilisant est un polymère bloc greffé ou séquencé, comprenant au moins un bloc résultant de la polymérisation de monomère à liaison(s) éthylénique(s) et au moins un bloc d'un polymère vinylique.

**11.** Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconé, et leurs mélanges.

**12.** Composition selon l'une des revendications précédentes, **caractérisée en ce que** la matière colorante comprend au moins un composé pulvérulent choisi parmi les charges, les pigments, les nacres et leurs mélanges.

**13.** Composition selon la revendication 12, **caractérisée en ce que** le composé pulvérulent et le polymère sont présents dans un rapport pigment(s)/polymère $\leq$ 0,9 et de préférence $\leq$ 0,5.

**14.** Composition selon la revendication 12 ou 13, **caractérisée en ce que** le composé pulvérulent représente jusqu'à 40 % du poids total de la composition.

**15.** Composition selon l'une des revendications 12 à 13, **caractérisée en ce que** le composé pulvérulent représente de 1 à 30 % du poids total de la composition.

**16.** Composition selon l'une des revendications précédentes, **caractérisée en ce que** le polymère représente (en matière sèche) jusqu'à 60 % du poids total de la composition.

**17.** Composition selon l'une des revendications précédentes, **caractérisée en ce que** le polymère représente (en matière sèche) de 12 à 60 % du poids total de la composition.

**18.** Composition selon l'une des revendications précédentes, **caractérisée en ce que** la phase grasse liquide contient au moins une huile choisie parmi les isoparaffines en $C_8$-$C_{16}$ et les silicones linéaires ou cycliques ayant de 2 à 7 atomes de silicium, ces silicones comportant éventuellement des groupes alkyle ayant de 1 à 10 atomes de carbone, leurs mélanges.

**19.** Composition selon l'une des revendications précédentes, dans laquelle la phase grasse liquide contient une huile volatile choisie parmi l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane, les isoparafines en $C_8$-$C_{16}$, et l'isododécane.

**20.** Composition selon l'une des revendications précédentes, se présentant sous forme d'un stick ou bâton ; sous la forme d'une pâte souple, de viscosité dynamique à 25°C de l'ordre de 1 à 40 Pa.s ; sous forme de coupelle ; de gel huileux ; de liquide huileux ; de dispersion vésiculaire contenant des lipides ioniques et/ou non ioniques.

**21.** Composition selon l'une des revendications précédentes, se présentant sous forme anhydre.

**22.** Composition selon l'une des revendications précédentes, se présentant sous la forme d'un produit de soin et/ou de maquillage de la peau et/ou des lèvres.

**23.** Composition selon l'une des revendications précédentes, se présentant sous forme d'un produit coulé.

**24.** Composition selon l'une des revendications précédentes, se présentant sous forme d'un fond de teint coulé, d'un fard à joues ou à paupières coulé, d'un rouge à lèvres, d'une base ou baume de soin pour les lèvres, d'un produit anti-cernes.

**25.** Composition selon l'une des revendications précédentes, **caractérisée en ce qu'**elle contient un actif choisi parmi les actifs cosmétiques, dermatologiques, hygiéniques ou pharmaceutiques.

**26.** Composition selon la revendication 25, **caractérisée en ce que** l'actif est choisi parmi les hydratants, vitamines, acides gras essentiels, sphingolipides, filtres solaires.

**27.** Utilisation dans une composition cosmétique ou pour la fabrication d'une composition pharmaceutique à application topique, comprenant une phase grasse liquide et au moins un ingrédient choisi parmi les matières colorantes et leurs mélanges, d'au moins 2 % en poids de particules de polymère filmifiable dispersées et stabilisées en surface par au moins un stabilisant dans ladite phase grasse liquide et d'au moins une huile volatile à température ambiante, ayant une tension de vapeur à température ambiante et pression atmosphériques allant de $10^{-3}$ à 300 mm de Hg à condition que la température d'ébullition soit supérieure à 30°C, la matière colorante contenant des pigments dans un rapport pigment(s)/polymère inférieur à 1, pour diminuer, voire supprimer, le transfert du film de composition déposé sur la peau et/ou les lèvres.

**28.** Utilisation dans une composition cosmétique ou pour la fabrication d'une composition pharmaceutique à application topique, d'au moins 2 % en poids, par rapport au poids total de la composition, de particules de polymère filmifiable dispersées et stabilisées en surface par au moins un stabilisant dans une phase grasse liquide et d'au moins une huile volatile à température ambiante, ayant une tension de vapeur à température ambiante et pression atmosphériques allant de $10^{-3}$ à 300 mm de Hg à condition que la température d'ébullition soit supérieure à 30°C pour diminuer, voire supprimer, le transfert du film de composition déposé sur les lèvres et/ou la peau d'être humain vers un support mis en contact avec le film, la composition contenant une matière colorante contenant des pigments dans un rapport pigment(s)/polymère inférieur à 1.

**29.** Procédé de soin cosmétique ou de maquillage des lèvres ou de la peau, consistant à appliquer sur respectivement les lèvres ou la peau une composition cosmétique telle définie aux revendications 1 à 26, les méthodes de traitement thérapeutique du corps humain étant exclues.

**30.** Procédé cosmétique pour limiter, voire supprimer, le transfert d'une composition de maquillage ou de soin cosmétique de la peau ou des lèvres sur un support différent de la peau ou des lèvres, contenant une phase grasse liquide et au moins un ingrédient choisi parmi les matières colorantes, consistant à introduire dans la phase grasse liquide au moins 2 % en poids, par rapport au poids total de la composition, de particules de polymère filmifiable dispersées et stabilisées en surface par au moins un stabilisant dans ladite phase grasse liquide, et au moins une huile volatile à température ambiante, ayant une tension de vapeur à température ambiante et pression atmosphériques allant de $10^{-3}$ à 300 mm de Hg à condition que la température d'ébullition soit supérieure à 30°C, la matière colorants contenant des pigments dans un rapport pigment(s)/polymère inférieur à 1.

**Patentansprüche**

**1.** Zusammensetzung zur topischen Anwendung, die eine flüssige Fettphase und mindestens ein Farbmittel enthält, **dadurch gekennzeichnet, daß** sie ferner mindestens 2 Gew.-% Partikel eines filmbildenden Polymers, bezogen auf das Gesamtgewicht der Zusammensetzung, enthält, die in der flüssigen Fettphase durch mindestens ein Stabilisierungsmittel an der Oberfläche stabilisiert und dispergiert sind, wobei die flüssige Fettphase mindestens ein bei Raumtemperatur flüchtiges Öl enthält, das bei Raumtemperatur und Atmosphärendruck einen Dampfdruck im Bereich von $10^{-3}$ bis 300 mm Hg aufweist, mit der Maßgabe, daß die Siedetemperatur über 30 °C liegt, und dadurch, daß das Farbmittel Pigmente in einem Verhältnis Pigment(e)/Polymer unter 1 enthält.

**2.** Zusammensetzung nach Anspruch 1, **dadurch gekennzeichnet, daß** die flüssige Fettphase eine kosmetische, dermatologische, hygienische oder pharmazeutische Fettphase ist.

**3.** Zusammensetzung nach einem der vorhergehenden Ansprüche, wobei das Polymer unter den durch radikalische Polymerisation hergestellten Polymeren, Polykondensaten, Polymeren natürlicher Herkunft und deren Gemischen ausgewählt ist.

**4.** Zusammensetzung nach einem der vorhergehenden Ansprüche, wobei das Polymer unter den Polyurethanen, Polyurethan-Acrylpolymeren, Polyharnstoffen, Polyharnstoff-Polyurethanen, Polyester-Polyurethanen, Polyether-Polyurethanen, Polyestern, Polyesteramiden, Polyestern mit Fettkette und Alkydharzen; Acryl- und/oder Vinylpolymeren oder Acryl- und/oder Vinylcopolymeren; Acryl-Silicon-Copolymeren; Polyacrylamiden; Siliconpolymeren, fluorierten Polymeren und deren Gemischen ausgewählt ist.

**5.** Zusammensetzung nach einem der vorhergehenden Ansprüche, **dadurch gekennzeichnet, daß** die flüssige Fettphase aus Ölen mineralischen, tierischen, pflanzlichen oder synthetischen Ursprungs, Ölen auf Kohlenstoffbasis, Kohlenwasserstoffölen, fluorierten Ölen und/oder Siliconölen oder Gemischen dieser Öle besteht.

**6.** Zusammensetzung nach einem der vorhergehenden Ansprüche, wobei die flüssige Fettphase ein Öl enthält, das unter Paraffinöl, Vaselineöl, Nerzöl, Schildkrötenöl, Sojaöl, Perhydrosqualen, Süßmandelöl, Calophyllumöl, Palmöl, Parleam, Traubenkernöl, Sesamöl, Maisöl, Rapsöl, Sonnenblumenöl, Baumwollöl, Aprikosenöl, Ricinusöl, Avocadoöl, Jojobaöl, Olivenöl oder Öl von Getreidekeimen; Estern von Lanolinsäure, Ölsäure, Laurinsäure und Stearinsäure; Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glycerintriisostearat oder Diglycerintriisostearat; höheren Fettsäuren, wie Myristinsäure, Palmitinsäure, Stearinsäure, Behensäure, Ölsäure, Linolsäure, Linolensäure oder Isostearinsäure; höheren Fettalkoholen, wie Cetanol, Stearylalkohol, Oleylalkohol, Linoleylalkohol, Linolenylalkohol, Isostearylalkohol oder Octyldodecanol; Siliconölen, wie PDMS, die gegebenenfalls Phenylgruppen enthalten, beispielsweise Phenyltrimethiconen, oder gegebenenfalls mit aliphatischen und/oder aromatischen Gruppen oder funktionellen Gruppen substituiert sind, wie Hydroxy-, Thiol- und/oder Aminogruppen; mit Fettsäuren, Fettalkoholen oder Polyoxyalkylenen modifizierten Polysiloxanen, fluorierten Siliconen und perfluorierten Ölen ausgewählt ist.

**7.** Zusammensetzung nach einem der vorhergehenden Ansprüche, wobei die flüssige Fettphase ausgewählt ist unter:

- nicht wäßrigen, flüssigen Verbindungen mit einem Gesamtsolubilitätsparameter nach dem Solubilitätsraum von HANSEN unter 17 $(MPa)^{1/2}$,
- Monoalkoholen mit einem Gesamtsolubilitätsparameter nach dem Solubilitätsraum von HANSEN von höchstens 20 $(MPa)^{1/2}$, oder
- deren Gemischen.

8. Zusammensetzung nach einem der vorhergehenden Ansprüche, wobei das Stabilisierungsmittel unter den Blockpolymeren, Pfropfpolymeren, statistischen Polymeren und deren Gemischen ausgewählt ist.

9. Zusammensetzung nach einem der vorhergehenden Ansprüche, wobei das Stabilisierungsmittel ausgewählt ist unter: den mit einer Kohlenwasserstoffkette gepfropften Siliconpolymeren; den mit einer Siliconkette gepfropften Kohlenwasserstoffpolymeren; den gepfropften Copolymeren, die ein unlösliches Grundgerüst vom Polyacryltyp mit löslichen Pfropfzweigen vom Polyhydroxystearinsäuretyp aufweisen; den gepfropften oder sequentiellen Blockcopolymeren, die mindestens einen Block vom Polyorganosiloxantyp und mindestens einen Block eines durch radikalische Polymerisation hergestellten Polymers enthalten; den gepfropften oder sequentiellen Blockcopolymeren, die mindestens einen Block vom Polyorganosiloxantyp und mindestens einen Polyetherblock aufweisen; den Copolymeren von Acrylaten oder Methacrylaten von $C_{1-4}$-Alkoholen und Acrylaten oder Methacrylaten von $C_{8-30}$-Alkoholen; den gepfropften oder sequentiellen Blockcopolymeren, die mindestens einen Block, der bei der Polymerisation von Monomeren mit einer oder mehreren ethylenischen Doppelbindungen gebildet wird, und mindestens einen Vinylpolymerblock enthalten; den gepfropften oder sequentiellen Blockcopolymeren, die mindestens einen Block, der bei der Polymerisation von Monomeren mit einer oder mehreren ethylenischen Doppelbindungen gebildet wird, und mindestens einen Acrylpolymerblock enthalten; und den gepfropften oder sequentiellen Blockcopolymeren, die mindestens einen Block, der bei der Polymerisation von Monomeren mit einer oder mehreren ethylenischen Doppelbindungen gebildet wird, und mindestens einen Polyetherblock enthalten.

10. Zusammensetzung nach einem der vorhergehenden Ansprüche, **dadurch gekennzeichnet, daß** das Stabilisierungsmittel ein gepfropftes oder sequentielles Blockcopolymer ist, das mindestens einen Block, der bei der Polymerisation von Monomeren mit einer oder mehreren ethylenischen Doppelbindungen gebildet wird, und mindestens einen Vinylpolymerblock enthält.

11. Zusammensetzung nach einem der vorhergehenden Ansprüche, die ferner mindestens eine zusätzliche Fettphase enthält, die unter den Wachsen, Gummen/Gummis und/oder pastösen Fettsubstanzen pflanzlichen, tierischen, mineralischen oder synthetischen Ursprungs oder auf Siliconbasis und deren Gemischen ausgewählt ist.

12. Zusammensetzung nach einem der vorhergehenden Ansprüche, **dadurch gekennzeichnet, daß** das Farbmittel pulverförmige Verbindungen umfasst, die unter den Füllstoffen und/oder Pigmenten und/oder Perlglanzpigmenten ausgewählt sind.

13. Zusammensetzung nach Anspruch 12, **dadurch gekennzeichnet, daß** die pulverförmige Verbindung und das Polymer in einem Verhältnis Pigment(e)/Polymer $\leq 9$ und vorzugsweise $\leq 5$ vorliegen.

14. Zusammensetzung nach Anspruch 12 oder 13, **dadurch gekennzeichnet, daß** die pulverförmige Verbindung bis zu 40 % des Gesamtgewichts der Zusammensetzung ausmacht.

15. Zusammensetzung nach einem der Ansprüche 12 bis 13, **dadurch gekennzeichnet, daß** die pulverförmige Verbindung 1 bis 30 % des Gesamtgewichts der Zusammensetzung ausmacht.

16. Zusammensetzung nach einem der vorhergehenden Ansprüche, **dadurch gekennzeichnet, daß** das Polymer (als Trockensubstanz) bis zu 60 % des Gesamtgewichts der Zusammensetzung ausmacht.

17. Zusammensetzung nach einem der vorhergehenden Ansprüche, **dadurch gekennzeichnet, daß** das Polymer (als Trockensubstanz) 12 bis 60 % des Gesamtgewichts der Zusammensetzung ausmacht.

18. Zusammensetzung nach einem der vorhergehenden Ansprüche, **dadurch gekennzeichnet, daß** die flüssige Fettphase mindestens ein Öl enthält, das unter den $C_{8-16}$-Isoparaffinen und den linearen oder cyclischen Siliconen mit 2 bis 7 Siliciumatomen, wobei die Silicone gegebenenfalls Alkylgruppen mit 1 bis 10 Kohlenstoffatomen aufweisen, oder deren Gemischen ausgewählt ist.

**19.** Zusammensetzung nach einem der vorhergehenden Ansprüche, wobei die flüssige Fettphase ein flüchtiges Öl enthält, das unter Octamethylcyclotetrasiloxan, Decamethylcyclopentasiloxan, Hexadecamethylcyclohexasiloxan, Heptamethylhexyltrisiloxan, Heptamethyloctyltrisiloxan, $C_{8-16}$-Isoparaffinen und Isododecan ausgewählt ist.

**20.** Zusammensetzung nach einem der vorhergehenden Ansprüche, die in Form eines Sticks oder Stiftes; in Form einer weichen Paste mit einer dynamischen Viskosität bei 25 °C in der Größenordnung von 1 bis 40 Pa·s; in Form eines Tiegelchens; als öliges Gel; als ölige Flüssigkeit; oder als Vesikeldispersion, die ionische und/oder nichtionische Lipide enthält, vorliegt.

**21.** Zusammensetzung nach einem der vorhergehenden Ansprüche, die in wasserfreier Form vorliegt.

**22.** Zusammensetzung nach einem der vorhergehenden Ansprüche, die in Form eines Produkts zur Pflege und/oder zum Schminken der Haut und/oder der Lippen vorliegt.

**23.** Zusammensetzung nach einem der vorhergehenden Ansprüche, die in Form eines gegossenen Produkts vorliegt.

**24.** Zusammensetzung nach einem der vorhergehenden Ansprüche, die in Form von gegossenem Make-up, als gegossenes Wangenrouge oder gegossener Lidschatten, als Lippenstift, als Grundmasse oder Pflegebalsam für die Lippen oder als Produkt gegen. Augenringe vorliegt.

**25.** Zusammensetzung nach einem der vorhergehenden Ansprüche, **dadurch gekennzeichnet, daß** sie einen kosmetischen, dermatologischen, hygienischen oder pharmazeutischen Wirkstoff enthält.

**26.** Zusammensetzung nach Anspruch 25, **dadurch gekennzeichnet, daß** der Wirkstoff unter den Hydratisierungsmitteln, Vitaminen, essentiellen Fettsäuren, Sphingolipiden und Sonnenschutzfiltern ausgewählt ist.

**27.** Verwendung von mindestens 2 Gew.-% Partikeln eines filmbildenden Polymers, die in einer flüssigen Fettphase durch mindestens ein Stabilisierungsmittel an der Oberfläche stabilisiert und dispergiert sind, und mindestens eines bei Raumtemperatur flüchtigen Öls, das bei Raumtemperatur und Atmosphärendruck einen Dampfdruck im Bereich von $10^{-3}$ bis 300 mm Hg aufweist, mit der Maßgabe, daß die Siedetemperatur über 30 °C liegt, wobei das Farbmittel Pigmente in einem Verhältnis Pigment(e) /Polymer unter 1 enthält, in einer kosmetischen Zusammensetzung oder zur Herstellung einer pharmazeutischen Zusammensetzung zur topischen Anwendung, die eine flüssige Fettphase und mindestens einen Bestandteil enthalten, der unter den Farbmitteln oder deren Gemischen ausgewählt ist, um das Übertragen eines Films einer auf der Haut und/oder den Lippen abgeschiedenen Zusammensetzung zu vermindern oder zu verhindern.

**28.** Verwendung von mindestens 2 Gew.-%, bezogen auf das Gesamtgewicht der Zusammensetzung, Partikeln eines filmbildenden Polymers, die in einer flüssigen Fettphase durch mindestens ein Stabilisierungsmittel an der Oberfläche stabilisiert und dispergiert sind, und mindestens eines bei Raumtemperatur flüchtigen Öls, das bei Raumtemperatur und Atmosphärendruck einen Dampfdruck im Bereich von $10^{-3}$ bis 300 mm Hg aufweist, mit der Maßgabe, daß die Siedetemperatur über 30 °C liegt, wobei die Zusammensetzung ein Farbmittel enthält, das Pigmente in einem Verhältnis Pigment(e)/Polymer unter 1 enthält, in einer kosmetischen Zusammensetzung oder zur Herstellung einer pharmazeutischen Zusammensetzung zur topischen Anwendung, um das Übertragen eines Films einer auf der Haut und/oder den Lippen abgeschiedenen Zusammensetzung auf einen Träger, der mit dem Film in Kontakt kommt, zu vermindern oder zu verhindern.

**29.** Verfahren zur kosmetischen Pflege oder zum Schminken der Lippen oder der Haut, das darin besteht, auf die Lippen und/oder die Haut eine kosmetische Zusammensetzung nach den Ansprüchen 1 bis 26 aufzubringen, wobei therapeutische Verfahren zur Behandlung des menschlichen Körpers ausgenommen sind.

**30.** Kosmetisches Verfahren, um das Übertragen einer kosmetischen Zusammensetzung zum Schminken oder zur Pflege der Haut oder der Lippen auf einen Träger, der von der Haut und den Lippen verschieden ist, zu vermindern oder zu verhindern, wobei die Zusammensetzung eine flüssige Fettphase und mindestens einen Bestandteil enthält, der unter den Farbmitteln ausgewählt ist, das darin besteht, in die flussige Fettphase mindestens 2 Gew.-%, bezogen auf das Gesamtgewicht der Zusammensetzung, Partikel eines filmbildenden Polymers, die in der flüssigen Fettphase durch mindestens ein Stabilisierungsmittel an der Oberfläche stabilisiert und dispergiert sind, und mindestens ein bei Raumtemperatur flüchtiges Öl einzuarbeiten, das bei Raumtemperatur und Atmosphärendruck im Bereich einen Dampfdruck von $10^{-3}$ bis 300 mm Hg aufweist, mit der Maßgabe, daß die Siedetemperatur über 30 °C liegt, wobei die Zusammensetzung ein Farbmittel enthält, das Pigmente in einem Verhältnis Pigment(e)/ Polymer unter 1 enthält.

**Claims**

1. Composition for topical application, comprising a liquid fatty phase and at least one dyestuff, **characterized in that** it also comprises at least 2% by weight, relative to the total weight of the composition, of polymer particles which can form a film and are dispersed and stabilized at the surface by at least one stabilizer, in the said liquid fatty phase, **in that** this fatty phase contains at least one oil that is volatile at ambient temperature, having a vapour pressure at ambient temperature and atmospheric pressure ranging from $10^{-3}$ to 300 mmHg, provided that the boiling point is above 30°C, and **in that** the dyestuff contains pigments in a pigment/polymer ratio of less than 1.

2. Composition according to Claim 1, **characterized in that** the liquid fatty phase is cosmetic, dermatological, hygienic or pharmaceutical.

3. Composition according to one of the preceding claims, in which the polymer is chosen from radical polymers, polycondensates and polymers of natural origin, and mixtures thereof.

4. Composition according to one of the preceding claims, in which the polymer is chosen from polyurethanes, polyurethane-acrylics, polyureas, polyurea/polyurethanes, polyester-polyurethanes, polyether-polyurethanes, polyesters, polyesteramides, polyesters containing a fatty chain, alkyds; acrylic and/or vinyl polymers or copolymers; acrylic-silicone copolymers; polyacrylamides; silicone polymers, fluoro polymers, and mixtures thereof.

5. Composition according to one of the preceding claims, in which the liquid fatty phase consists of carbon-based, hydrocarbon-based, fluoro and/or silicone oils of mineral, animal, plant or synthetic origin, alone or as a mixture.

6. Composition according to one of the preceding claims, in which the liquid fatty phase contains an oil which is chosen from liquid paraffin, liquid petroleum jelly, mink oil, turtle oil, soybean oil, perhydrosqualene, sweet almond oil, beauty-leaf oil, palm oil, parleam oil, grapeseed oil, sesame oil, corn oil, rapeseed oil, sunflower oil, cottonseed oil, apricot oil, castor oil, avocado oil, jojoba oil, olive oil or cereal germ oil; esters of lanolic acid, of oleic acid, of lauric acid or of stearic acid; fatty esters, such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, di(2-ethylhexyl) succinate, diisostearyl malate, glyceryl triisostearate or diglyceryl triisostearate; higher fatty acids, such as myristic acid, palmitic acid, stearic acid, behenic acid, oleic acid, linoleic acid, linolenic acid or isostearic acid; higher fatty alcohols, such as ketanol, stearyl alcohol or oleyl alcohol, linoleyl or linolenyl alcohol, isostearyl alcohol or octyldodecanol; silicone oils, such as PDMSs, which are optionally phenylated, such as phenyltrimethicones, or which are optionally substituted with aliphatic and/or aromatic groups or with functional groups such as hydroxyl, thiol and/or amine groups; polysiloxanes modified with fatty acids, fatty alcohols or polyoxyalkylenes, fluorosilicones and perfluoro oils.

7. Composition according to one of the preceding claims, in which the liquid fatty phase is chosen from the group comprising:

   - non-aqueous liquid compounds with a global solubility parameter, according to the Hansen solubility space, of less than 17 $(MPa)^{1/2}$,

   - or monoalcohols with a global solubility parameter, according to the Hansen solubility space, of less than or equal to 20 $(MPa)^{1/2}$,

   - or mixtures thereof.

8. Composition according to one of the preceding claims, in which the stabilizer is chosen from block polymers, grafted polymers and random polymers, and mixtures thereof.

9. Composition according to one of the preceding claims, in which the stabilizer is chosen from silicone polymers grafted with a hydrocarbon-based chain; hydrocarbon-based polymers grafted with a silicone chain; grafted copolymers having an insoluble skeleton of polyacrylic type with soluble grafts of polyhydroxystearic acid type; block or grafted block copolymers comprising at least one block of polyorganosiloxane type and at least one block of a radical polymer; block or grafted block copolymers comprising at least one block of polyorganosiloxane type and at least one block of a polyether; copolymers of $C_1$-$C_4$ alcohol acrylates or methacrylates or of $C_8$-$C_{30}$ alcohol acrylates or methacrylates; block or grafted block copolymers comprising at least one block resulting from the polymerization of a monomer containing (an) ethylenic bond(s) and at least one block of a vinyl polymer; block or grafted block copolymers comprising at least one block resulting from the polymerization of a monomer containing (an) ethylenic bond(s) and at least one block of an acrylic polymer; block or grafted block copolymers comprising at least one block resulting from the polymerization of a monomer containing (an) ethylenic bond(s) and at least one block of a polyether.

10. Composition according to one of the preceding claims, **characterized in that** the stabilizer is a block or grafted block polymer comprising at least one block resulting from the polymerization of a monomer containing (an) ethylenic bond(s) and at least one block of a vinyl polymer.

11. Composition according to one of the preceding claims, also comprising at least one additional fatty phase chosen from waxes, gums and/or pasty fatty substances of plant, animal, mineral or synthetic origin, or which are silicone-based, and mixtures thereof.

12. Composition according to one of the preceding claims, **characterized in that** the dyestuff comprises at least one pulverulent compound chosen from fillers, pigments and pearlescent agents, and mixtures thereof.

13. Composition according to Claim 12, **characterized in that** the pulverulent compound and the polymer are present in a pigment/polymer ratio of $\leq 0.9$ and preferably $\leq 0.5$.

14. Composition according to Claim 12 or 13, **characterized in that** the pulverulent compound represents up to 40% of the total weight of the composition.

15. Composition according to either of Claims 12 and 13, **characterized in that** the pulverulent compound represents from 1 to 30% of the total weight of the composition.

16. Composition according to one of the preceding claims, **characterized in that** the polymer represents (as solids) up to 60% of the total weight of the composition.

17. Composition according to one of the preceding claims, **characterized in that** the polymer represents (as solids) from 12 to 60% of the total weight of the composition.

18. Composition according to one of the preceding claims, **characterized in that** the liquid fatty phase contains at least one oil chosen from $C_8$-$C_{16}$ isoparaffins and linear or cyclic silicones containing from 2 to 7 silicon atoms, these silicones optionally including alkyl groups containing from 1 to 10 carbon atoms, and mixtures thereof.

19. Composition according to one of the preceding claims, in which the liquid fatty phase contains a volatile oil chosen from octamethylcyclotetrasiloxane, decamethylcyclopentasiloxane, hexadecamethylcyclohexasiloxane, heptamethylhexyltrisiloxane, heptamethyloctyltrisiloxane, $C_8$-$C_{16}$ isoparaffins and isododecane.

20. Composition according to one of the preceding claims, which is in the form of a stick or tube; in the form of a supple paste with a dynamic viscosity at 25°C of about 1 to 40 Pa.s; in the form of a dish; an oily gel; an oily liquid; a vesicle dispersion containing ionic and/or nonionic lipids.

21. Composition according to one of the preceding claims, which is in anhydrous form.

22. Composition according to one of the preceding claims, which is in the form of a care product and/or a make-up product for the skin and/or the lips.

23. Composition according to one of the preceding claims, which is in the form of a cast product.

24. Composition according to one of the preceding claims, which is in the form of a cast foundation, a cast blusher or eye shadow, a lipstick, a lipcare balm or base or a concealer product.

25. Composition according to one of the preceding claims, **characterized in that** it contains an active agent chosen from cosmetic, dermatological, hygiene and pharmaceutical active agents.

26. Compositien according to Claim 25, **characterized in that** the active agent is chosen from moisturizers, vitamins, essential fatty acids, sphingolipids and sunscreens.

27. Use, in a cosmetic composition or for the manufacture of a pharmaceutical composition for topical application, comprising a liquid fatty phase and at least one ingredient chosen from dyestuffs and mixtures thereof, of at least 2% by weight of film-forming polymer particles which are dispersed and surface-stabilized with at least one stabilizer in the said liquid fatty phase, and of at least one oil that is volatile at ambient temperature, having a vapour pressure at ambient temperature and atmospheric pressure ranging from $10^{-3}$ to 300 mmHg, provided that the boiling point is greater than 30°C, the dyestuff containing pigments in a pigment/polymer ratio of less than 1, to reduce, or even eliminate altogether, the transfer of the film of composition deposited on the skin and/or the lips.

28. Use, in a cosmetic composition or for the manufacture of a pharmaceutical composition for topical application, of at least 2% by weight, relative to the total weight of the composition, of film-forming polymer particles which are dispersed and surface-stabilized with at least one stabilizer in a liquid fatty phase, and of at least one oil that is volatile at ambient temperature, having a vapour pressure at ambient temperature and atmospheric pressure ranging from $10^{-3}$ to 300 mmHg, provided that the boiling point is greater than 30°C, to reduce, or even eliminate altogether, the transfer of the film of composition deposited on the lips and/or the skin of a human being onto a support placed in contact with the film, the composition containing a dyestuff containing pigments in a pigment/polymer ratio of less than 1.

29. Cosmetic process to care for or make up the lips or the skin, which consists in applying a cosmetic composition as defined in Claims 1 to 26 to the lips or the skin, respectively, excluding methods for the therapeutic treatment of the human body.

30. Cosmetic process for limiting, or even eliminating altogether, the transfer of a make-up or cosmetic care composition from the skin or the lips onto a support other than the skin or the lips, this composition containing a liquid fatty phase and at least one ingredient chosen from dyestuffs, this process consisting in introducing into the liquid fatty phase at least 2% by weight, relative to the total weight of the composition, of polymer particles which can form a film and are dispersed and stabilized at the surface by at least one stabilizer in the said liquid fatty phase, and at least one oil that is volatile at ambient temperature, having a vapour pressure at ambient temperature and atmospheric pressure ranging from $10^{-3}$ to 300 mmHg, provided that the boiling point is greater than 30°C, the dyestuff containing pigments in a pigment/polymer ratio of less than 1.